# EUROPEAN PATENT SPECIFICATION

(11) **EP 4 020 055 B1**
(45) Date of publication and mention of the grant of the patent: **10.09.2025**
(21) Application number: 20858757.6
(22) Date of filing: 17.08.2020
(51) Int. Cl.: G02B 23/24, G01B 11/02, A61B 1/00, A61B 1/04, A61B 1/06

(54) **ENDOSCOPE DEVICE, OPERATING METHOD THEREFOR, AND PROGRAM FOR ENDOSCOPE DEVICE**
ENDOSKOPVORRICHTUNG, BETRIEBSVERFAHREN DAFÜR UND PROGRAMM FÜR ENDOSKOPVORRICHTUNG
DISPOSITIF ENDOSCOPE, SON PROCÉDÉ DE FONCTIONNEMENT ET PROGRAMME POUR DISPOSITIF ENDOSCOPE

(30) Priority: 23.08.2019 JP 2019152839
(43) Date of publication of application: 29.06.2022
(73) Proprietor: FUJIFILM Corporation, Tokyo 106-8620 (JP)
(72) Inventor: YOSHIOKA, Masato, Kanagawa 258-8538 (JP); TATSUTA, Takeichi, Kanagawa 258-8538 (JP)
(74) Representative: Dehns Germany Partnerschaft mbB
(86) International application number: PCT/JP2020/031013
(87) International publication number: WO 2021/039471

(56) References cited:
- EP-A1- 3 571 976
- WO-A1-2018/051680
- WO-A1-2018/055933
- WO-A1-2018/180249
- WO-A1-2019/017018
- JP-A- 2008 125 996
- JP-A- 2014 160 109
- JP-A- H08 228 999
- US-A1- 2020 107 698

## Description

### BACKGROUND OF THE INVENTION

### 1. Field of the Invention

The present invention relates to an endoscope apparatus that measures the size of a subject, a method of operating the endoscope apparatus, and a program for an endoscope apparatus.

### 2. Description of the Related Art

In an endoscope apparatus, a distance to a subject is measured or the length or size of the subject is calculated. In, for example, WO2018/051680A, a subject is irradiated with auxiliary measurement light and a spot is formed on the subject. The position of the spot is specified from a picked-up image that is obtained from the image pickup of the subject. Then, an index figure, which shows the actual size of an object to be observed included in the subject, is set according to the position of the spot, and a measurement marker consisting of the set index figure is displayed in the picked-up image. Since the measurement marker includes scales that extend vertically and horizontally, the size of the object to be observed can be measured in a case where the measurement marker to be displayed is matched with the object to be observed. The size of the object to be observed can be measured using the measurement marker displayed in the picked-up image in this way. An endoscope apparatus is also disclosed in WO2019/017018.

### SUMMARY OF THE INVENTION

The surface of a subject of which the image is to be picked up by an endoscope apparatus may have a three-dimensional shape, such an uneven shape. Since a measurement marker having a size different from the actual size of the object to be observed is displayed in a case where the spot formed by the auxiliary measurement light is formed on the three-dimensional shape, an error may be generated. The reason for this is that a difference in a distance to the spot in the depth direction along an optical axis is generated between a position at which the spot is formed on the three-dimensional shape and the position of the spot in a case where there is no portion having a three-dimensional shape, such as a uneven shape, that is, the position of the spot at the base of the three-dimensional shape. In particular, in a case where the object to be observed is closely observed in a state where the object to be observed is moved to the center of the picked-up image in order to observe or diagnose the object to be observed in detail, the spot is often formed on the three-dimensional shape. Accordingly, in a case where the object to be observed is to be observed in detail, an error may be generated on the scale displayed according to the position of the spot.

On the other hand, a method of displaying a scale, which is offset with a predicted error in a state where the three-dimensional shape of a subject or an object to be observed is defined in advance, is conceivable. However, there is a concern that a definition for offset is also complicated since an actual uneven shape varies.

The present invention has been made in consideration of the above-mentioned circumstances, and an object of the present invention is to provide an endoscope apparatus that displays a more accurate scale on an object to be observed, a method of operating the endoscope apparatus, and a program for an endoscope apparatus.

An endoscope apparatus according to the invention is defined in claim 1.

It is preferable that the measurement marker has a shape of a straight line segment or a shape in which straight line segments are combined with each other.

It is preferable that the gradations of the measurement marker are uneven.

It is preferable that the processor creates the specific image in which the measurement marker is superimposed on the picked-up image so that a center of the measurement marker is positioned closer to a center than the position of the specific region.

It is preferable that the auxiliary measurement light source unit emits the auxiliary measurement light so that a trajectory formed by the auxiliary measurement light and a trajectory formed by an optical axis of the image pickup element are different from each other on the subject.

It is preferable that the processor receives an instruction to switch the measurement marker to be set and switches and sets a plurality of the measurement markers, which are different from each other, according to the instruction.

It is preferable that the processor switches the measurement marker that includes the gradations of which the end portion serves as a base point to set a measurement marker that includes gradations of which a middle portion serves as a base point.

It is preferable that the endoscope apparatus further comprises an illumination light source unit emitting illumination light used to illuminate the subject and an illumination light switch used to turn on or off the illumination light, and the auxiliary measurement light source unit does not emit the auxiliary measurement light in a case where the illumination light is turned off by the illumination light switch.

Furthermore, a program for an endoscope apparatus according to invention is defined in claim 9.

According to the present invention, a more accurate scale can be displayed on an object to be observed.

### BRIEF DESCRIPTION OF THE DRAWINGS

Fig. 1 is a diagram showing the appearance of an endoscope apparatus.
Fig. 2 is a plan view of a distal end part of an endoscope.
Fig. 3 is a block diagram showing the functions of the endoscope apparatus.
Fig. 4 is a block diagram showing the functions of an auxiliary measurement light-emitting unit.
Fig. 5 is a diagram illustrating a spot SP that is formed on a subject by auxiliary measurement light.
Fig. 6 is a diagram illustrating a relationship between the distal end part of the endoscope and a near end Px, an intermediate vicinity Py, and a far end Pz in a range Rx of an observation distance and a relationship between an optical axis and auxiliary measurement light.
Fig. 7 is a block diagram showing the functions of a signal processing unit.
Fig. 8 is an image diagram of a picked-up image in which the spot SP is formed.
Fig. 9 is an image diagram of an example of a specific image in which a measurement marker is superimposed on a picked-up image.
Fig. 10 is an image diagram of an example of a specific image in which a measurement marker is superimposed on a picked-up image.
Fig. 11 is an image diagram of an example of a specific image in which a measurement marker is superimposed on a picked-up image.
Figs. 12A, 12B, and 12C are diagrams showing types of measurement markers, Fig. 12A shows a measurement marker that includes a line segment and gradations on the left side of the spot SP, Fig. 12B shows a measurement marker that includes a line segment and gradations on the lower side of the spot SP, and Fig. 12C shows a measurement marker that includes a line segment and gradations on the upper right side of the spot SP.
Fig. 13 is a diagram illustrating measurement markers having a cruciform shape with gradations, a distorted cruciform shape, a circular-and-cruciform shape, and the shape of a measurement point group.
Fig. 14 is a diagram illustrating a relationship between auxiliary measurement light and an observation distance with regard to a subject having a three-dimensional shape.
Fig. 15 is an image diagram of an example of a specific image in which a measurement marker is superimposed on a picked-up image.
Fig. 16 is a diagram illustrating the position of the measurement marker in the picked-up image.
Fig. 17 is a diagram illustrating trajectories that are formed on a subject by illumination light and auxiliary measurement light.
Fig. 18 is a diagram illustrating the switching of a measurement marker.
Fig. 19 is a flowchart illustrating a flow of displaying a specific image by the endoscope apparatus.

### DESCRIPTION OF THE PREFERRED EMBODIMENTS

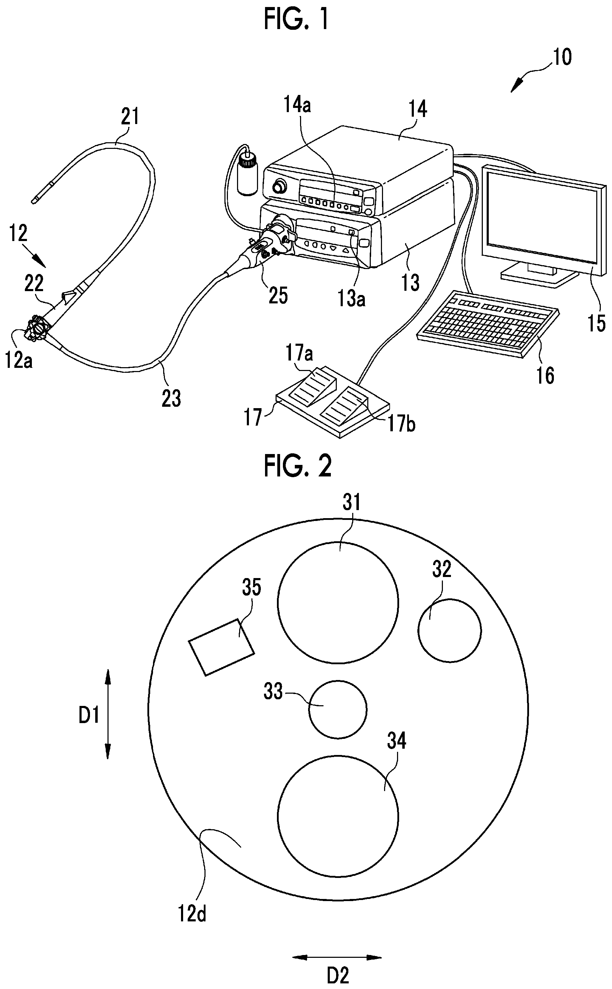

As shown in Fig. 1, an endoscope apparatus 10 includes an endoscope 12, a light source device 13, a processor device 14, a monitor 15, and a keyboard 16 and a foot switch 17 that are a user interface. The endoscope 12 is optically connected to the light source device 13, and is electrically connected to the processor device 14. The processor device 14 is electrically connected to the monitor 15 (display unit) that displays an image. The keyboard 16 and the foot switch 17, which are a user interface, are connected to the processor device 14, and are used for various setting operations and the like for the processor device 14. The user interface includes a mouse and the like in addition to the keyboard 16 or the foot switch 17 shown in Fig. 1.

The processor device 14 includes processor buttons 14a that are used to give preset various instructions. The processor buttons 14a may be installed at a part of an operation panel, such as a touch panel, connected to the processor device 14. Further, the light source device 13 includes a light source button 13a that is used to give preset various instructions.

The endoscope 12 includes an insertion part 21 that is to be inserted into an object to be examined, an operation part 22 that is provided at the proximal end portion of the insertion part 21, and a universal cable 23. The operation part 22 includes a scope button 12a that is used to give preset various instructions while a user of the endoscope 12 is operating the endoscope 12. The universal cable 23 is a cable in which a light guide part (not shown) for guiding illumination light emitted from the light source device 13, a control line for transmitting control signals used to control the endoscope 12, a signal line for transmitting image signals obtained from the image pickup of an object to be observed, a power line for supplying power to each part of the endoscope 12, and the like are integrated. The distal end of the universal cable 23 is provided with a connector 25 to be connected to the light source device 13. Further, the light guide part of the endoscope 12 is a light guide in which optical fibers are bundled.

The endoscope 12 has a normal mode and a length measurement mode, and these two modes are switched by an instruction. A mode switching instruction can be set in any one or more of the processor buttons 14a, the scope button 12a, the foot switch 17, or the like. These buttons function as a mode changeover switch by setting.

The normal mode is a mode in which a picked-up image obtained from the image pickup of an object to be observed illuminated with illumination light is displayed. Accordingly, a measurement marker is not displayed in the normal mode. The length measurement mode is a mode in which an object to be observed is illuminated with illumination light and auxiliary measurement light and a measurement marker used for the measurement of the size and the like of the object to be observed is displayed in a picked-up image obtained from the image pickup of the object to be observed. The auxiliary measurement light is light that is used for the measurement of the object to be observed.

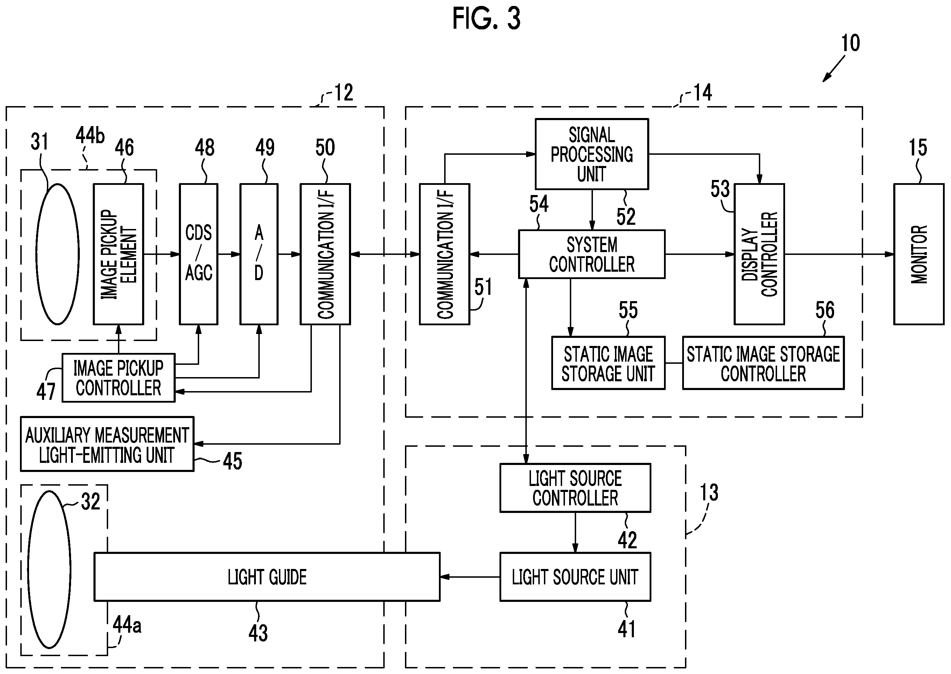

The function of a static image-acquisition instruction switch, which gives an instruction to acquire the static image of a picked-up image, may be set in any one or more of the processor buttons 14a, the scope button 12a, the foot switch 17, or the like (static image-acquisition instruction unit). In a case where a user gives an instruction to acquire a static image by the static image-acquisition instruction switch, the screen of the monitor 15 is frozen and displayed and an alert sound (for example, "beep") informing the acquisition of a static image is generated together. Then, for example, the static images of the picked-up image, which are obtained before and after the operation timing of the scope button 12a, are stored in a static image storage unit 55 (Fig. 3) provided in the processor device 14. Further, it is preferable that measurement information to be described later is also stored together with the static image of the picked-up image in a case where the endoscope 12 is set to the length measurement mode. The static image storage unit 55 is a storage unit, such as a hard disk or a universal serial bus (USB) memory. In a case where the processor device 14 can be connected to a network, the static image of the picked-up image may be stored in a static image storage server (not shown), which is connected to the network, instead of or in addition to the static image storage unit 55.

Further, a gesture recognition unit (not shown), which recognizes the gestures of a user, may be connected to the processor device 14, and may be adapted to give a static image-acquisition instruction in a case where the gesture recognition unit recognizes a specific gesture of a user. The gesture recognition unit may also be used for the switching of a mode, and the like.

Furthermore, a sight line input unit (not shown), which is provided close to the monitor 15, may be connected to the processor device 14, and may be adapted to give a static image-acquisition instruction in a case where the sight line input unit recognizes that a user's sight line is in a predetermined region of the monitor 15 for a predetermined time or longer. Moreover, a voice recognition unit (not shown) may be connected to the processor device 14, and may be adapted to give a static image-acquisition instruction in a case where the voice recognition unit recognizes a specific voice generated by a user. The voice recognition unit may also be used for the switching of a mode, and the like.

As shown in Fig. 2, a distal end part 12d of the endoscope 12 has a substantially circular shape, and is provided with an objective lens 31 that is positioned closest to a subject among optical members of an image pickup optical system of the endoscope 12, an illumination lens 32 that is used to irradiate the subject with illumination light, an auxiliary measurement lens 33 that is used to illuminate the subject with auxiliary measurement light to be described later, an opening 34 that allows a treatment tool to protrude toward the subject, and an air/water supply nozzle 35 that is used to supply air and water.

An optical axis Ax of the image pickup optical system 44b (Fig. 3) extends in a direction perpendicular to the plane of paper. A vertical first direction D1 is orthogonal to the optical axis Ax, and a horizontal second direction D2 is orthogonal to the optical axis Ax and the first direction D1. The objective lens 31 and the auxiliary measurement lens 33 are arranged in the first direction D1.

As shown in Fig. 3, the light source device 13 comprises a light source unit 41 and a light source controller 42. The light source unit 41 (illumination light source unit) generates illumination light that is used to illuminate the subject. The illumination light, which is emitted from the light source unit 41, is incident on a light guide 43, and the subject is irradiated with the illumination light through the illumination lens 32. A white light source emitting white light, a plurality of light sources, which include a white light source and a light source emitting another color light (for example, a blue light source emitting blue light), or the like is used as a light source of the illumination light in the light source unit 41. In this embodiment, the function of an illumination light switch, which is used to turn on or off the illumination light, is set in the above-mentioned light source button 13a.

An illumination optical system 44a, an image pickup optical system 44b, and an auxiliary measurement light-emitting unit (auxiliary measurement light source unit) 45 are provided in the distal end part 12d of the endoscope. The illumination optical system 44a includes the illumination lens 32, and the object to be observed is irradiated with light, which is emitted from the light guide 43, through the illumination lens 32. The image pickup optical system 44b includes the objective lens 31 and an image pickup element 46. Light reflected from the object to be observed is incident on the image pickup element 46 through the objective lens 31. Accordingly, the reflected image of the object to be observed is formed on the image pickup element 46. The auxiliary measurement light-emitting unit 45 emits auxiliary measurement light that is used for the measurement of the subject.

The image pickup element 46 is a color image pickup sensor, and picks up the reflected image of the subject and outputs image signals. It is preferable that the image pickup element 46 is a charge coupled device (CCD) image pickup sensor, a complementary metal-oxide semiconductor (CMOS) image pickup sensor, or the like. The image pickup element 46 used in the embodiment of the present invention is a color image pickup sensor that is used to obtain RGB image signals corresponding to three colors of R (red), G (green), and B (blue). The image pickup element 46 is controlled by an image pickup controller 47.

Image signals output from the image pickup element 46 are transmitted to a CDS/AGC circuit 48. The CDS/AGC circuit 48 performs correlated double sampling (CDS) or auto gain control (AGC) on the image signals that are analog signals. The image signals, which have been transmitted through the CDS/AGC circuit 48, are converted into digital image signals by an analog/digital converter (A/D converter) 49. The digital image signals, which have been subjected to A/D conversion, are input to the processor device 14 through a communication interface (I/F) 50.

The processor device 14 comprises a communication I/F 51 that is connected to the communication I/F 50 of the endoscope 12, a signal processing unit 52, a display controller 53, a system controller 54, the static image storage unit 55, and a static image storage controller 56. Programs related to the signal processing unit 52, the display controller 53, the system controller 54, the static image storage unit 55, the static image storage controller 56, and the like are incorporated into a memory (not shown) of the processor device 14. The programs are operated by the system controller 54 formed of a processor, so that the functions of the signal processing unit 52, the display controller 53, the system controller 54, the static image storage unit 55, the static image storage controller 56, and the like are realized.

The communication I/F 51 receives the image signals, which are transmitted from the communication I/F 50 of the endoscope 12, and transmits the image signals to the signal processing unit 52. A memory, which temporarily stores the image signals received from the communication I/F 51, is built in the signal processing unit 52, and the signal processing unit 52 processes an image signal group, which is a set of the image signals stored in the memory, to create the picked-up image. In a case where the endoscope 12 is set to the length measurement mode, the signal processing unit 52 may be adapted to perform structure-emphasis processing for emphasizing structures, such as blood vessels, or color difference-emphasis processing for increasing a color difference between a normal area and a lesion area of the object to be observed on the picked-up image.

The display controller 53 causes the monitor 15 to display the picked-up image, which is created by the signal processing unit 52, or a specific image. The system controller 54 performs the control of the image pickup element 46 through the image pickup controller 47 provided in the endoscope 12. The image pickup controller 47 also performs the control of the CDS/AGC circuit 48 and the A/D converter 49 together with the control of the image pickup element 46. The static image storage controller 56 performs a control related to the static image of the picked-up image stored in the static image storage unit 55.

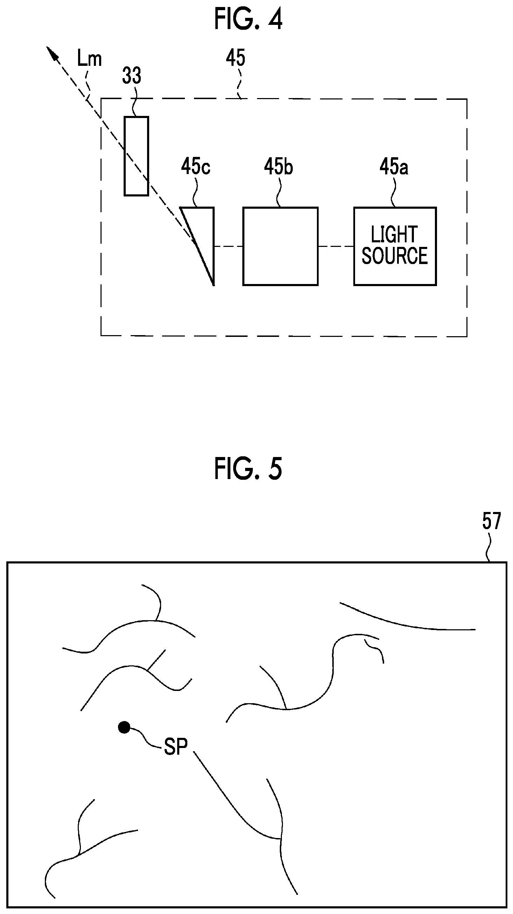

As shown in Fig. 4, the auxiliary measurement light-emitting unit 45 comprises a light source 45a, an auxiliary measurement light-generating element 45b, a prism 45c, and the auxiliary measurement lens 33. The light source 45a emits the auxiliary measurement light. The light source 45a that emits auxiliary measurement light used for the measurement of the subject, is to emit light having a color that can be detected by pixels of the image pickup element 46 (specifically visible light), and includes a light emitting element, such as a laser light source (laser diode (LD)) or a light emitting diode (LED), and a condenser lens that condenses light emitted from the light emitting element.

It is preferable that light emitted from the light source 45a is red light having a wavelength in the range of, for example, 600 nm to 650 nm. Alternatively green light having a wavelength in the range of 495 nm to 570 nm may be used. The auxiliary measurement light-generating element 45b converts light, which is emitted from the light source, into the auxiliary measurement light that is used to obtain measurement information. In order to convert the light into the auxiliary measurement light, the auxiliary measurement light-generating element 45b specifically uses a collimator lens, a diffractive optical element (DOE), or the like.

The prism 45c is an optical member that is used to change the travel direction of the auxiliary measurement light converted by the auxiliary measurement light-generating element 45b. The prism 45c changes the travel direction of the auxiliary measurement light so that the auxiliary measurement light intersects with the visual field of the image pickup optical system including the objective lens 31 and a lens group. The details of the travel direction of the auxiliary measurement light will be described later. A subject is irradiated with auxiliary measurement light Lm, which is emitted from the prism 45c, through the auxiliary measurement lens 33.

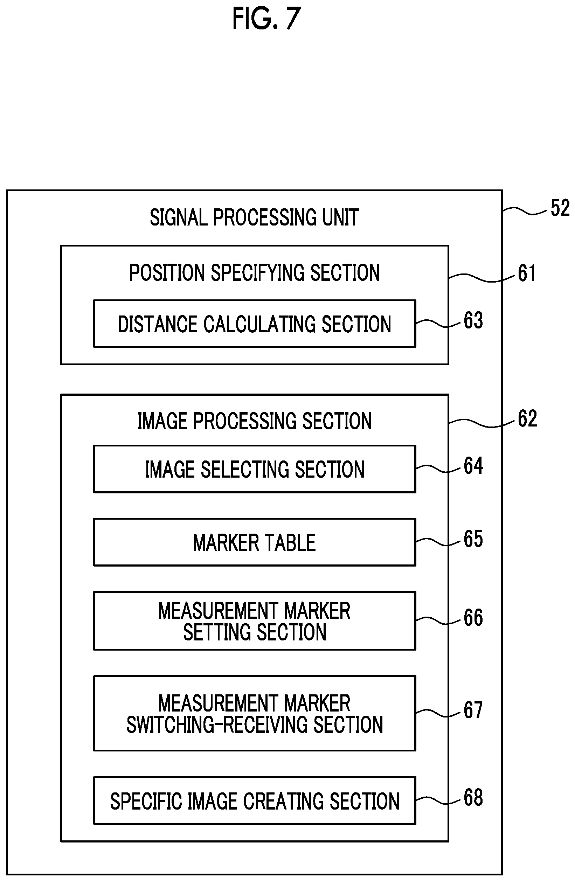

The subject is irradiated with the auxiliary measurement light, so that a specific region is formed on the subject as shown in Fig. 5. According to the invention, the specific region is a spot SP and is a circular region. The communication I/F 51, which is an image acquisition unit, acquires a picked-up image 57 obtained from the image pickup of the subject which is illuminated with the illumination light and on which the spot SP is formed by the auxiliary measurement light. The position of the spot SP in the picked-up image, which is acquired by the communication I/F 51, is specified by a position specifying section 61 (Fig. 7). An observation distance, which is a distance between the distal end part 12d of the endoscope and the subject, is obtained from the specified position of the spot SP. A measurement marker showing an actual size is set according to this observation distance. The set measurement marker is displayed in the picked-up image.

An auxiliary measurement slit, which is formed at the distal end part 12d of the endoscope, may be used instead of the auxiliary measurement lens 33. Further, it is preferable that an anti-reflection coating (AR coating) (anti-reflection portion) is provided on the auxiliary measurement lens 33. The reason why the anti-reflection coating is provided as described above is that it is difficult for the position specifying section 61 (Fig. 7) to recognize the position of the spot SP formed on the subject by the auxiliary measurement light in a case where the auxiliary measurement light is reflected without being transmitted through the auxiliary measurement lens 33 and a ratio of the auxiliary measurement light with which a subject is irradiated is reduced.

The auxiliary measurement light-emitting unit 45 has only to be capable of emitting the auxiliary measurement light to the visual field of the image pickup optical system. For example, the light source 45a may be provided in the light source device and light emitted from the light source 45a may be guided to the auxiliary measurement light-generating element 45b by optical fibers. Further, the prism 45c may not be used and the orientations of the light source 45a and the auxiliary measurement light-generating element 45b may be inclined with respect to the optical axis Ax of the image pickup optical system 44b so that the auxiliary measurement light is emitted in a direction crossing the visual field of the image pickup optical system.

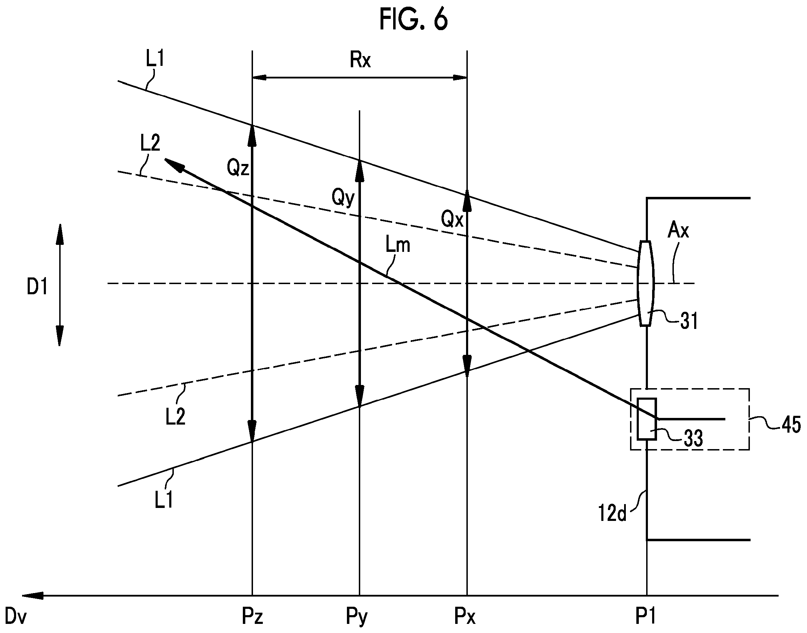

With regard to the travel direction of the auxiliary measurement light, the auxiliary measurement light is emitted in a state where the optical axis of the auxiliary measurement light Lm is in the image pickup angle of view (in a region between two solid lines L1) of the image pickup optical system as shown in Fig. 6. In a case where the subject can be observed in a range Rx of the observation distance, it is understood that the positions (points where the respective arrows Qx, Qy, and Qz intersect with the optical axis of the auxiliary measurement light Lm) of the spot SP, which is formed on the subject by the auxiliary measurement light, in image pickup ranges (shown by arrows Qx, Qy, and Qz) at a near end Px, an intermediate vicinity Py, and a far end Pz of the range Rx are different from each other. The position of the distal end part 12d of the endoscope is defined as a position P1. The observation distance is a distance between the distal end part 12d of the endoscope and the subject. Accordingly, the observation distance is a distance between the position P1 and each of the near end Px, the intermediate vicinity Py, and the far end Pz. In detail, the observation distance is a distance between the starting point of the optical axis Ax of the image pickup optical system 44b of the distal end part 12d of the endoscope and the subject. An axis Dv represents the observation distance. The image pickup angle of view of the image pickup optical system is represented by the region between the two solid lines L1, and measurement is performed in a central region (a region between two dotted lines L2), in which an aberration is small, of this image pickup angle of view.

Since the auxiliary measurement light is emitted in a state where the optical axis of the measurement light Lm is in the image pickup angle of view of the image pickup optical system as described above, the sensitivity of the movement of the position of the spot to a change in the observation distance is high. Accordingly, the size of the subject can be measured with high accuracy. The image of the subject illuminated with the auxiliary measurement light is picked up by the image pickup element 46, so that a picked-up image including the spot SP is obtained. In the picked-up image, the position of the spot SP varies depending on a relationship between the optical axis Ax of the image pickup optical system 44b and the optical axis of the auxiliary measurement light Lm and the observation distance. However, the number of pixels showing the same actual size (for example, 5 mm) is increased in the case of a short observation distance, and the number of pixels showing the same actual size is reduced in the case of a long observation distance.

Accordingly, in a case where information, which represents a relationship between the position of the spot SP and measurement information (the number of pixels) corresponding to the actual size of the subject, is stored in advance, measurement information can be calculated from the position spot SP.

As shown in Fig. 7, the signal processing unit 52 of the processor device 14 comprises a position specifying section 61 and an image processing section 62 to recognize the position of the spot SP, to calculate an observation distance to the subject, and to set various measurement markers. The position specifying section 61 specifies the position of the spot SP in the picked-up image, and calculates the observation distance. The image processing section 62 sets various measurement markers on the basis of the observation distance, and creates a specific image, which is obtained from the processing of the picked-up image using the various measurement markers. The specific image is caused to be displayed on the monitor 15 by the display controller 53.

In a case where the endoscope 12 is set to the length measurement mode, the light source unit 41 and the auxiliary measurement light-emitting unit 45 continuously emit the illumination light and the auxiliary measurement light. In some cases, the auxiliary measurement light may be turned on or dimmed to be emitted. The picked-up image is an RGB image corresponding to three colors, but may be other color images (brightness signal Y and color difference signals Cr and Cb). Accordingly, in a case where the endoscope 12 is set to the length measurement mode, the picked-up image of the subject illuminated with the illumination light and the auxiliary measurement light is input to the signal processing unit 52. The picked-up image is acquired by the communication I/F 51 (image acquisition unit).

In a case where the endoscope 12 is set to the normal mode, the light source unit 41 constantly emits the illumination light. The subject is irradiated with the illumination light through the light guide 43. Since the light source 45a of the auxiliary measurement light-emitting unit 45 is stopped in the case of the normal mode, the auxiliary measurement light is turned off. Accordingly, in a case where the endoscope 12 is set to the normal mode, the picked-up image of the subject illuminated with the illumination light is input to the signal processing unit 52. The picked-up image is acquired by the communication I/F 51 (image acquisition unit).

The position specifying section 61 specifies the position of the spot SP that is formed on the subject by the auxiliary measurement light. Specifying the position of the spot SP is performed on the basis of the picked-up image of the subject that is illuminated with the illumination light and the auxiliary measurement light in the length measurement mode. With regard to the picked-up image, the image of the subject on which the spot SP is formed by the auxiliary measurement light is acquired through the image pickup optical system and the image pickup element. Accordingly, the position specifying section 61 specifies the position of the spot SP on the image pickup element on the basis of the image of the subject. Further, the position specifying section 61 includes a distance calculating section 63. The distance calculating section 63 can obtain the observation distance from the position of the spot SP.

The image processing section 62 includes an image selecting section 64, a marker table 65, a measurement marker setting section 66, a measurement marker switching-receiving section 67, and a specific image creating section 68. The image selecting section 64 selects the picked-up image obtained in the length measurement mode, which is an image to be subjected to processing based on the position of the spot SP, between the picked-up image obtained in the normal mode and the picked-up image obtained in the length measurement mode. The marker table 65 is a table in which information showing a relationship between the position of the spot SP corresponding to an observation distance and the measurement information (the number of pixels) corresponding to the actual size of the subject is stored in advance. The measurement marker setting section 66 sets a measurement marker that shows the actual size of an object to be observed on the subject according to the position of the spot SP and includes gradations of which an end portion serves as a base point. The measurement marker switching-receiving section 67 receives an instruction to switch and set a plurality of measurement markers. The specific image creating section 68 creates a specific image in which the measurement marker set by the measurement marker setting section 66 is superimposed on a picked-up image so that the position of the spot SP and the base point of the gradations of the measurement marker overlap with each other.

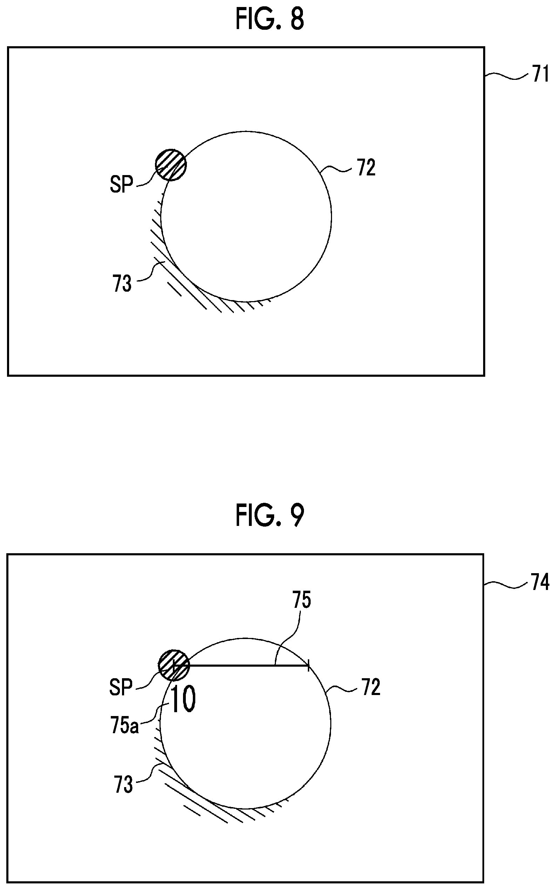

The functions of the measurement marker setting section 66 and the specific image creating section 68 will be specifically described below. As shown in Fig. 8, a picked-up image 71 of which a subject including a polyp 72, which is an object to be observed, is illuminated with the illumination light and the auxiliary measurement light is input to the signal processing unit 52 in the length measurement mode. For example, since the polyp 72 has a three-dimensional spherical shape, the picked-up image 71 includes the polyp 72 and the spot SP and includes a shadow 73 in some cases.

The position specifying section 61 specifies the position of the spot SP on the basis of the picked-up image 71 input to the signal processing unit 52. The measurement marker setting section 66 sets a measurement marker, which shows the actual size of the object to be observed corresponding to the position of the spot SP and includes gradations of which an end portion serves as a base point, with reference to the marker table 65. The end portion is a portion closer to the outer portion than a central portion, or a starting point, an end point, or the like in the shape of the measurement marker.

As shown in Fig. 9, the specific image creating section 68 creates a specific image 74 in which a measurement marker 75 set by the measurement marker setting section 66 is superimposed on a picked-up image 71 so that the position of the spot SP and the base point of the gradations of the measurement marker 75 overlap with each other. It is preferable that the measurement marker 75 is superimposed to be displayed at the position of the spot SP for more accurate measurement. Accordingly, it is preferable that the measurement marker 75 is displayed close to the spot SP as much as possible even in a case where the measurement marker 75 is displayed at a position away from the spot SP. The measurement marker 75 is a straight line segment, and includes gradations, which are line segments perpendicular to the straight line segment, at the starting point and the end point of the line segment. In a case where the measurement marker 75 is a line segment or the like and has a starting point and an end point, the starting point and/or the end point themselves may be used as gradations. In this case, for example, gradations having the shape of a line segment perpendicular to the straight line segment may not be provided. Further, the measurement marker 75 may include a number "10" near the base point of the gradations. This is a gradation label 75a of the measurement marker 75, and is given so that the line segment of the measurement marker 75 can be easily recognized as an actual size of 10 mm. Hereinafter, numbers included in measurement markers have the same meaning. The numerical value of the gradation label 75a can be changed by setting, and a measurement marker 75 of which a gradation label 75a itself is not displayed may be provided.

Various types of measurement markers are used depending on settings. For example, a measurement marker having the shape of a straight line segment or a shape in which straight line segments are combined with each other, a measurement marker having a circular shape or a shape in which circles are combined with each other, or a measurement marker having a shape in which a circle and a straight line segment are combined with each other, and the like are used.

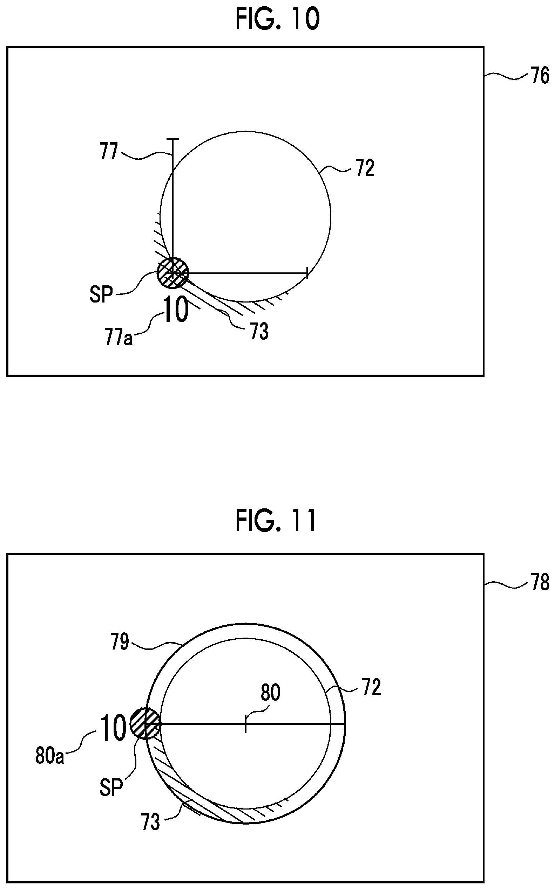

As shown in Fig. 10, for example, a specific image 76 includes a measurement marker 77 having a shape in which straight line segments are combined with each other. The measurement marker 77 has a shape in which straight line segments are combined in an L shape, the line segments extend upward along the plane of paper and to the right along the plane of paper from the corner of the L shape as a base point, and the measurement marker 77 includes a gradation at each of end points of the line segments with the base point as a starting point. Further, the measurement marker 77 includes a number "10", which is a gradation label 77a, near the base point of the gradations like the measurement marker 75.

As shown in Fig. 11, for example, a specific image 78 includes a measurement marker 79 having a shape in which a straight line segment and a circle are combined with each other. The measurement marker 79 has a shape in which a circle and a line segment corresponding to the diameter of the circle are combined with each other, and the line segment extends to the right along the plane of paper from one of intersections between the line segment and the circle as a base point. With regard to the line segment or the circle, the respective intersections between the line segment and the circle are used as gradations. The measurement marker 79 may include a gradation 80 at a point where the line segment is divided in half or the center of the circle. Further, the measurement marker 79 includes a number "10", which is a gradation label 79a, near the base point of the gradations like the measurement marker 75 or the measurement marker 77.

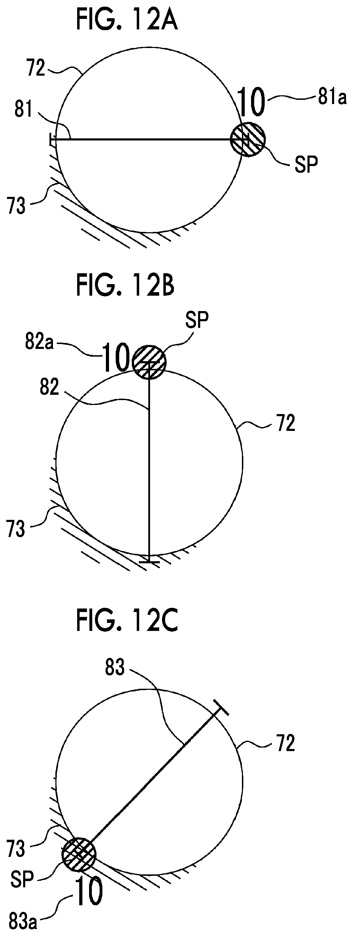

As shown in Figs. 12A, 12B, and 12C, in addition to these, the measurement marker may have various shapes like, for example, a measurement marker 81 (Fig. 12A) of which a line segment extends from a base point to the left along the plane of paper and which includes a gradation label 81a, a measurement marker 82 (Fig. 12B) of which a line segment extends downward along the plane of paper from a base point and which includes a gradation label 82a, a measurement marker 83 (Fig. 12C) of which a line segment extends in a direction oblique to the upper right side along the plane of paper from a base point and which includes a gradation label 83a, or the like.

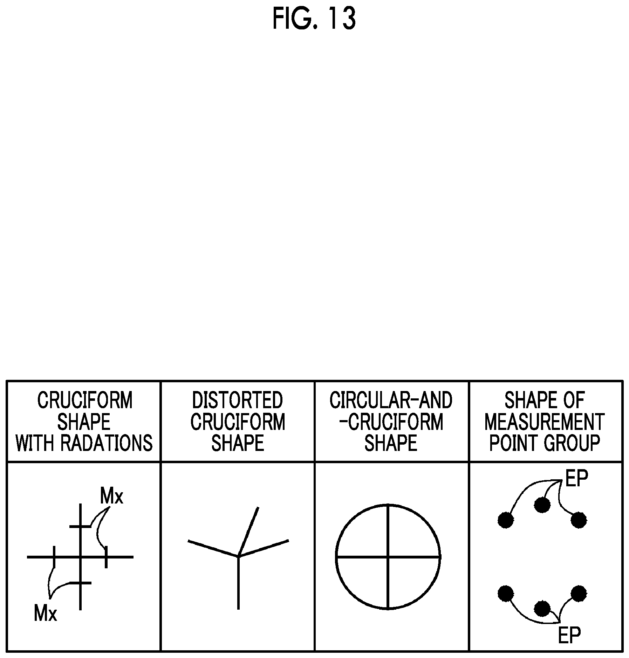

In addition, as shown in Fig. 13, the measurement marker may have a cruciform shape with gradations where gradations Mx are given to at least one of a vertical line or a horizontal line of a cruciform shape. Further, the measurement marker may have a distorted cruciform shape of which at least one of a vertical line or a horizontal line is inclined. Furthermore, the measurement marker may have a circular-and-cruciform shape where a cruciform shape and a circle are combined with each other. In these cases, the gradations can be provided from each end portion of the cruciform shape to the intersection of the cruciform shape. In addition, the measurement marker may have the shape of a measurement point group where a plurality of measurement points EP corresponding to an actual size from the spot SP are combined with each other. In this case, distances between the spot SP and the measurement points EP can be used as gradations.

Further, one measurement marker may be displayed or a plurality of measurement markers may be displayed, and the color of the measurement marker may be changed according to an actual size. Furthermore, with regard to the size of the measurement marker, a measurement marker having a size corresponding to an object to be observed may be displayed or a measurement marker having a size smaller or larger than the object to be observed may be displayed, and the actual size of the measurement marker may be set to any value (for example, 2 mm, 3 mm, 10 mm, or the like) according to the object to be observed or the purpose of observation.

The gradations of the measurement marker may be uneven. For example, in a case where an object to be observed having a three-dimensional shape is measured, the measurement marker setting section 66 sets a measurement marker that is distorted in consideration of the three-dimensional shape of the object to be observed. The consideration of the three-dimensional shape is performed by the estimation of the height of a three-dimensional shape using a distance to the object to be observed, which is calculated by the distance calculating section 63, or the estimation of the size or shape of a three-dimensional body using the image analysis of the picked-up image. A machine learning technique, which uses a learned model having learned the picked-up image, may be used in the image analysis.

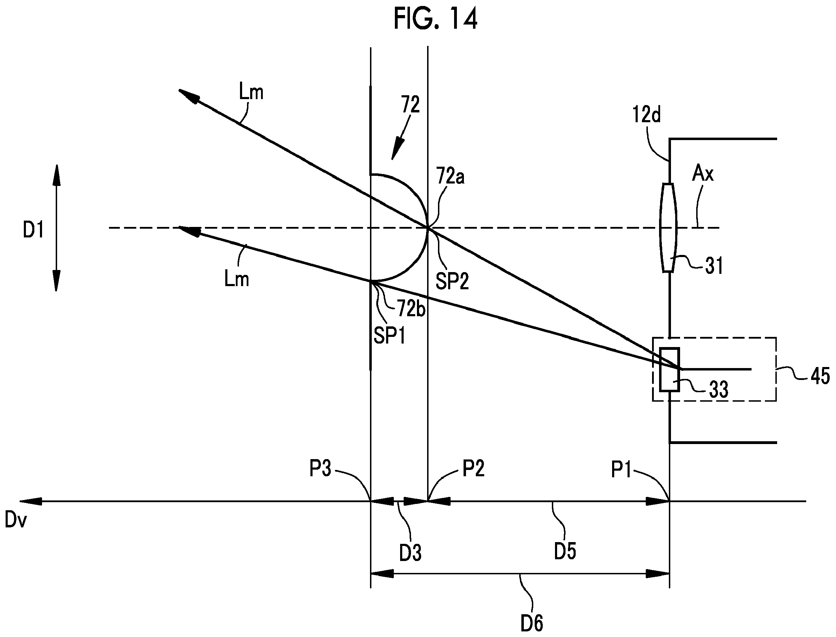

For example, as shown in Fig. 14, a spot SP2 is formed at an apex 72a of the polyp 72 by the auxiliary measurement light Lm. A distance to the object to be observed, which is calculated from the spot SP2, is a distance D5 between the position P1 of the distal end part 12d of the endoscope and a position P2 of the spot SP2. Further, the spot SP1 is formed at an end portion 72b of the polyp 72 by the auxiliary measurement light Lm. A distance to the object to be observed, which is calculated from the spot SP1, is a distance D6 between the position P1 of the distal end part 12d of the endoscope and a position P3 of the spot SP1. Accordingly, the height of the polyp 72 is a distance D3 that is a difference between the distances D6 and D5.

The measurement marker setting section 66 stores, for example, the distances D6 and D5 to calculate the distance D3 and sets the distance D3 as the height of the object to be observed. Further, information about the size and/or shape of the object to be observed, which is estimated by the image analysis of an observation image or the like, is also used to grasp a three-dimensional shape. The measurement marker setting section 66 sets a measurement marker that is distorted in consideration of the three-dimensional shape of the object to be observed grasped in this way.

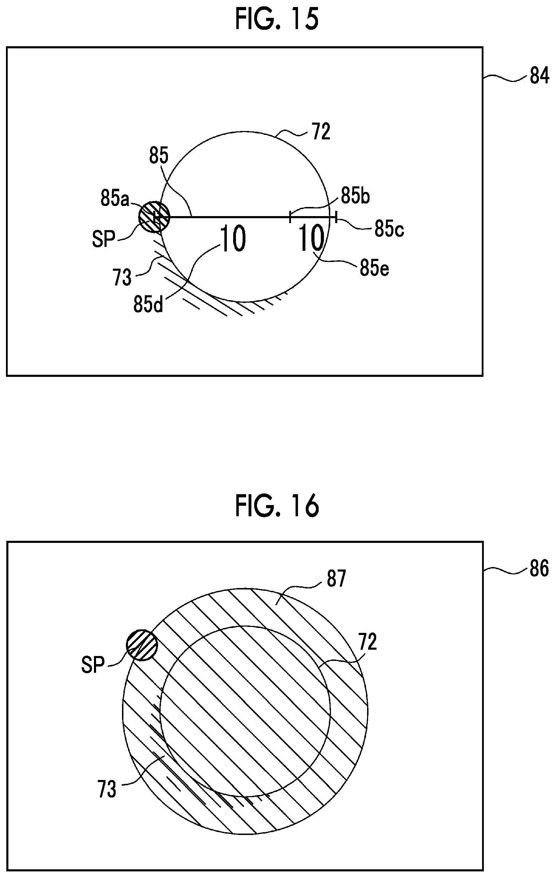

For example, as shown in Fig. 15, the measurement marker setting section 66 sets a measurement marker 85, which is distorted in consideration of the three-dimensional shape of the polyp 72, on the basis of information about the height, size, and shape of the polyp 72, and the like. The measurement marker 85 includes a gradation 85a, a gradation 85b, and a gradation 85c. The gradation 85a is positioned at the starting point of the measurement marker 85, and the gradation 85c is positioned at the end point of the measurement marker 85. The gradation 85b is positioned closer to the end point than the middle of the measurement marker 85. Further, a number "10", which is a gradation label 85d, means that a measured size from the gradation 85a to the gradation 85b is 10 mm, and a number "10", which is a gradation label 85e, means that a measured size from the gradation 85b to the gradation 85c is 10 mm. A distance between the gradations 85a and 85b and a distance between the gradations 85b and 85c in the measurement marker 85 are not equal to each other in the observation image, but the gradations of the measurement marker 85 show the actual size since the actual polyp 72 has a spherical shape. The measurement marker setting section 66 sets the measurement marker 85, and the specific image creating section 68 superimposes the measurement marker 85 on the picked-up image to create a specific image 84.

As described above, the measurement marker setting section 66 sets a measurement marker that shows an actual size according to the position of the spot SP and includes gradations of which an end portion serves as a base point, and the specific image creating section 68 creates a specific image in which the measurement marker set by the measurement marker setting section 66 is superimposed on a picked-up image so that the position of the spot SP and the base point of the gradations of the measurement marker overlap with each other. In many cases, it may be natural that a user positions an object to be observed which the user wants to measure at the center of a picked-up image obtained from the endoscope for more detailed observation and observes the object to be observed in detail. Accordingly, since the specific image in which the measurement marker is superimposed as described above is created, the position of the measurement marker can be more appropriately disposed. Therefore, for example, a more accurate scale can be displayed on the object to be observed as compared to a case where the measurement marker is disposed at the peripheral portion of the picked-up image.

Further, the object to be observed which the user wants to measure may have a three-dimensional shape. Accordingly, since the specific image in which the measurement marker is superimposed as described above is created, an error in which a value measured using the measurement marker is smaller than an actual distance to the object to be observed or the size of the object to be observed is measured to be smaller than the actual size of the object to be observed is reduced in a case where the spot SP is formed at the apex portion of the three-dimensional shape. As a result, a measurement marker closer to the actual distance can be displayed. Therefore, a more accurate scale can be displayed on the object to be observed. The underestimation of the size of the object to be observed, for example, a polyp can be prevented by the above-mentioned structure and a more appropriate diagnosis can be made.

It is preferable that the specific image creating section 68 creates a specific image in which a measurement marker is superimposed on a picked-up image so that the center of the measurement marker is positioned closer to a center than the position of the spot SP. In many cases, it may be natural that a user positions an object to be observed which the user wants to measure at the center of a picked-up image obtained from the endoscope for more detailed observation and observes the object to be observed in detail. Accordingly, since the specific image in which the measurement marker is disposed closer to the center than the spot SP is created, the measurement marker can be more appropriately disposed.

For example, as shown in Fig. 16, the center of a measurement marker is positioned in a region 87, which is a region closer to the central region of the specific image 86 than the position of the spot SP, in a specific image 86. Accordingly, the position of the measurement marker can be more appropriately disposed. The region 87 is a region shown in Fig. 16 by diagonal lines. Therefore, for example, a more accurate scale can be displayed on the object to be observed as compared to a case where the measurement marker is disposed at the peripheral portion of the picked-up image. The peripheral portion of the picked-up image can be a region of the specific image 86 other than the region 87.

Further, it is preferable that the auxiliary measurement light-emitting unit 45 emits the auxiliary measurement light so that a trajectory formed by the auxiliary measurement light and a trajectory formed by the optical axis of the image pickup element (the optical axis of the image pickup optical system) are different from each other on the subject. Furthermore, a fact that the trajectories are different from each other means that the trajectories do not coincide with each other. Accordingly, since the object to be observed is positioned close to the center of the picked-up image and the spot SP is formed closer to the outside than the vicinity of the optical axis of the image pickup optical system, the object to be observed can be disposed at the center and the object to be observed is easily observed.

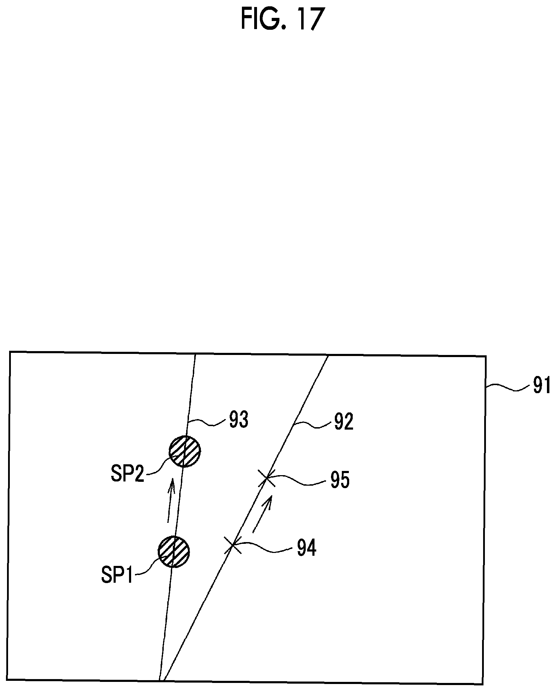

For example, the auxiliary measurement light is adjusted so that a spot SP1 formed by the auxiliary measurement light is positioned on the left side of an observation point 94 in the picked-up image 91, in a picked-up image 91 in which a trajectory 93 formed by the auxiliary measurement light and a trajectory 92 formed by the optical axis of the image pickup optical system are recorded on the subject as shown in Fig. 17, in a case where the optical axis of the image pickup optical system is positioned at the observation point 94. Then, the auxiliary measurement light is adjusted so that a spot SP2 formed by the auxiliary measurement light is positioned on the left side of an observation point 95 in the picked-up image 91 in a case where the observation point is moved and the optical axis of the image pickup optical system is positioned at the observation point 95. It is preferable that the trajectory 93 formed by the auxiliary measurement light is positioned on the outside of a trajectory formed on the subject by the optical axis of the image pickup optical system, and the trajectory 93 may be positioned on the left or right side or on the upper or lower side. Accordingly, since the measurement marker is disposed closer to the center of the picked-up image than the spot SP, a more accurate scale can be displayed on the object to be observed.

The measurement markers are switched and displayed as desired. The measurement marker switching-receiving section 67 receives an instruction to switch the measurement marker to be set. The measurement marker setting section 66 switches and sets a plurality of measurement markers, which are different from each other, according to the instruction of the measurement marker switching-receiving section 67. As described above, markers having various shapes can be set as the measurement marker that shows an actual size and includes gradations of which an end portion serves as a base point. Accordingly, in a case where an instruction to switch the setting of the measurement marker in advance is assigned to any one or more of, for example, the processor buttons 14a, the scope button 12a, the foot switch 17, or the like, a user can create a specific image while switching a measurement marker to the most suitable measurement marker during observation.

Further, the measurement marker setting section may switch the measurement marker that includes gradations of which an end portion serves as a base point, and set a measurement marker that includes gradations of which a middle portion serves as a base point. Even in a case where the measurement marker that includes gradations of which a middle portion serves as a base point is set, it is preferable to create a specific image in which the measurement marker is superimposed on a picked-up image so that the spot SP and the base point of the gradations of the measurement marker overlap with each other.

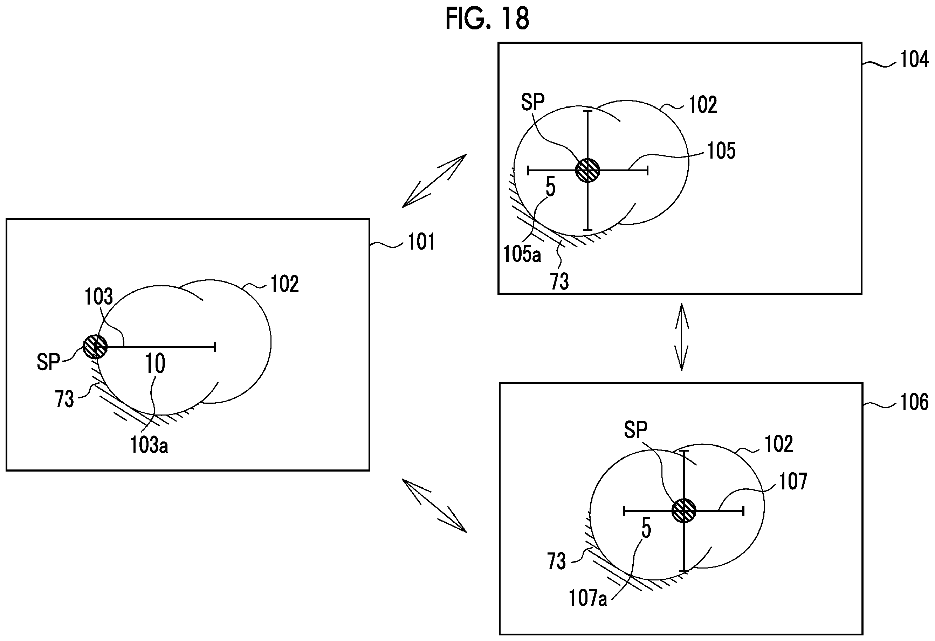

In a case where a polyp 102 having the shape of two connected spheres is set as an object to be observed in a specific image 101 as shown in Fig. 18, a measurement marker 103 is superimposed on a picked-up image so that a spot SP and an end portion of the measurement marker 103 overlap with each other. Since a gradation label 103a of gradations of the measurement marker 103 is displayed as "10", the length of the measurement marker from a starting point to an end point is 10 mm that is a measured size. Here, a user operates, for example, the foot switch 17, which is the measurement marker switching-receiving section 67, to measure the entire length of the polyp 102. The foot switch 17 includes a left switch 17a and a right switch 17b (Fig. 1). The left switch 17a is a changeover switch that is used to circulate, switch, and display a plurality of different measurement markers one after the other. The plurality of measurement markers also include a measurement marker that includes gradations of which a middle portion serves as a base point in addition to a measurement marker that includes gradations of which an end portion serves as a base point. The right switch 17b is a confirmation switch, and a user operates the right switch 17b by stepping on the right switch 17b with a foot in a case where a desired measurement marker is displayed.

For example, a case where the specific image 101 is switched to a specific image 104 is a case where the measurement marker 103 is switched to a measurement marker 105. In this case, the position of the spot SP is not changed and a measurement marker is switched to the measurement marker 105 from the measurement marker 103. Since the position of the spot SP is not changed, a user shifts the image pickup optical system to the right so that the polyp 102 can be measured. Since the measurement marker is a cruciform marker, line segments extend vertically and horizontally from the center of the measurement marker, and a gradation label 105a is displayed as "5", each of the line segments shows a measured size of 5 mm. Accordingly, the size or length of the polyp 102 can be measured and grasped.

Further, a case where the specific image 101 is switched to a specific image 106 is a case where the measurement marker 103 is switched to a measurement marker 107. In this case, the position of the spot SP is changed and is moved to the central portion of the picked-up image. Accordingly, an object to be observed can be observed, imaged, or measured at the center of the picked-up image. Since a gradation label 107a is displayed as "5" in the specific image 106, it is found that the shorter length of the three-dimensional shape of the polyp 102 having the shape of two connected spheres is about 10 mm.

Furthermore, not only the switching of the specific image 101 to the specific image 104 but also the switching of the specific image 104 to the specific image 101 can be performed by the measurement marker switching-receiving section 67. Moreover, not only the switching of the specific image 101 to the specific image 106 but also the switching of the specific image 106 to the specific image 101 can be performed likewise. Further, not only the switching of the specific image 104 to the specific image 106 but also the switching of the specific image 106 to the specific image 104 can be performed. Furthermore, the measurement marker may be switched to measurement markers having the same shape and different gradations in addition to the switching of the measurement markers having different shapes. Accordingly, a measurement marker, which is considered as a more appropriate measurement marker by a user, can be easily set as desired.

It is preferable that the auxiliary measurement light-emitting unit 45 does not emit the auxiliary measurement light in a case where the illumination light is turned off by an illumination light switch that is used to turn on or off the illumination light used to illuminate a subject. As described above, the light source button 13a (Fig. 1) of the light source device 13 (illumination light source unit) has a function of the illumination light switch. In a case where the illumination light is turned off by the light source button 13a, such as a case where the endoscope is not present in the body, the auxiliary measurement light-emitting unit 45 does not emit the auxiliary measurement light. Since the auxiliary measurement light is laser light in this embodiment, it is preferable in terms of safety that the auxiliary measurement light is not turned on in a case where the endoscope is present outside the body. However, in a case where an interlock function of the auxiliary measurement light is given to the illumination light switch, the unintended emission of the auxiliary measurement light can be prevented even though an instruction to turn on the auxiliary measurement light is given in a case where the illumination light is turned off by the illumination light switch. Since the auxiliary measurement light is turned on in a case where a mode is switched to the length measurement mode, the emission of the auxiliary measurement light can be prevented as described above in a case where, for example, the endoscope is present outside the body and the illumination light is turned off by the illumination light switch even though any one or more of the processor buttons 14a, the scope button 12a, the foot switch 17, or the like in which a mode switching instruction is set are unintentionally operated.

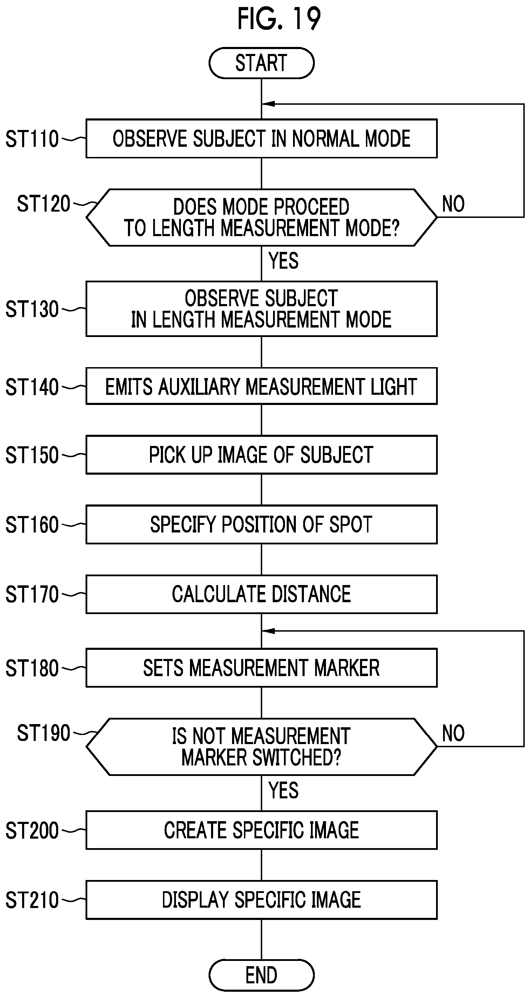

Next, the action of the above configuration will be described with reference to the flowchart of Fig. 19. First, a subject is observed in the normal mode (Step ST110). For example, in a case where an object to be observed, which needs to be observed and measured, is found in the subject, a mode proceeds to the length measurement mode (YES in Step ST120). In a case where a mode does not proceed to the length measurement mode (NO in Step ST120), the subject continues to be observed in the normal mode.

In a case where a mode proceeds to the length measurement mode, the subject is observed in the length measurement mode (Step ST130). The auxiliary measurement light-emitting unit 45 emits the auxiliary measurement light (Step ST140), and a picked-up image of the subject is acquired by the image pickup element (Step ST150). The position specifying section 61 specifies the position of the spot SP on the basis of the picked-up image (Step ST160). In some cases, the distance calculating section 63 calculates a distance between the distal end part 12d of the endoscope and the subject (Step ST170) and transmits the calculated distance to the measurement marker setting section 66 or the like.

The measurement marker setting section 66 sets a measurement marker using the obtained information (Step ST180). The measurement marker setting section 66 sets a measurement marker that shows an actual size according to the position of the spot SP specified by the position specifying section 61 and includes gradations of which an end portion serves as a base point. In a case where the measurement marker is switched (NO in Step ST190), the process returns to a step in which the measurement marker is not yet set. In a case where the measurement marker is not switched (YES in Step ST190), the process proceeds to the creation of a specific image. A specific image in which the measurement marker is superimposed on the picked-up image so that the position of the spot SP and the base point of the gradations of the measurement marker overlap with each other is created as the specific image (Step ST200). After the specific image is created, the specific image is displayed on the monitor 15 (Fig. 1) or the like in some cases (Step ST210).

In the embodiment, the hardware structure of a processing unit, which performs various types of processing, such as the signal processing unit 52, the display controller 53, or the system controller 54, is various processors to be described below. Various processors include: a central processing unit (CPU) that is a general-purpose processor functioning as various processing units by executing software (program); a programmable logic device (PLD) that is a processor of which the circuit configuration can be changed after manufacture, such as a field programmable gate array (FPGA); a dedicated electrical circuit that is a processor having circuit configuration designed exclusively to perform various types of processing; and the like.

One processing unit may be formed of one of these various processors, or may be formed of a combination of two or more same kind or different kinds of processors (for example, a plurality of FPGAs, or a combination of a CPU and an FPGA). Further, a plurality of processing units may be formed of one processor. As an example where a plurality of processing units are formed of one processor, first, there is an aspect where one processor is formed of a combination of one or more CPUs and software as typified by a computer, such as a client or a server, and functions as a plurality of processing units. Second, there is an aspect where a processor fulfilling the functions of the entire system, which includes a plurality of processing units, by one integrated circuit (IC) chip as typified by System On Chip (SoC) or the like is used. In this way, various processing units are formed using one or more of the above-mentioned various processors as hardware structures.

In addition, the hardware structures of these various processors are more specifically electrical circuitry where circuit elements, such as semiconductor elements, are combined. Another aspect of the present invention is an endoscope apparatus that comprises an auxiliary measurement light source unit emitting auxiliary measurement light used for the measurement of a subject and an image pickup element picking up an image of the subject. A processor specifies the position of a specific region formed on the subject by the auxiliary measurement light, sets a measurement marker that shows an actual size according to the position of the specified specific region and includes gradations of which an end portion serves as a base point, and creates a specific image in which the measurement marker is superimposed on the picked-up image of the subject picked up by the image pickup element so that the position of the specific region and the base point of the gradations of the measurement marker overlap with each other.

It goes without saying that the present invention is not limited to the embodiment and can employ various configurations without departing from the scope of the present invention as defined by the claims. In addition, the present invention covers not only a program but also a storage medium storing a program.

### Explanation of References

10: endoscope apparatus
12: endoscope
12a: scope button
12d: distal end part
13: light source device
13a: light source button
14: processor device
14a: processor button
15: monitor
16: keyboard
17: foot switch
17a: left switch
17b: right switch
21: insertion part
22: operation part
23: universal cable
25: connector
31: objective lens
32: illumination lens
33: auxiliary measurement lens
34: opening
35: air/water supply nozzle
41: light source unit
42: light source controller
43: light guide
44a: illumination optical system
44b: image pickup optical system
45: auxiliary measurement light-emitting unit
45a: light source
45b: auxiliary measurement light-generating element
45c: prism
46: image pickup element
47: image pickup controller
48: CDS/AGC circuit
49: A/D converter
50, 51: communication I/F
52: signal processing unit
53: display controller
54: system controller
55: static image storage unit
56: static image storage controller
57, 91: picked-up image
61: position specifying section
62: image processing section
63: distance calculating section
64: image selecting section
65: marker table
66: measurement marker setting section
67: measurement marker switching-receiving section
68: specific image creating section
71, 74, 76, 78, 84, 86, 101, 104, 106: specific image
72, 102: polyp
72a: apex
72b: end portion
73: shadow
75, 77, 79, 81, 82, 83, 85, 103, 105, 107: measurement marker
75a, 77a, 80a, 81a, 82a, 83a, 85d, 85e, 103a, 105a, 107a: gradation label
85a, 85b, 85c: gradation
87: region
92, 93: trajectory
94, 95: observation point
L1: solid line
L2: dotted line
D1: first direction
D2: second direction
D3: height of spot SP1
D5, D6: observation distance
Dv: observation distance
Lm: auxiliary measurement light
Ax: optical axis of image pickup optical system
Px: near end
Py: intermediate vicinity
Pz: far end
P1 to P3: position
Qx, Qy, Qz: image pickup range
Rx: range of observation distance
SP, SP1, SP2: spot
EP: measurement point
Mx: gradation
ST110 to ST210: Step

## Claims

1. An endoscope apparatus comprising:
an auxiliary measurement light source unit (45) that emits auxiliary measurement light used for measurement of a subject;
an image pickup element (46) that picks up an image of the subject; wherein the auxiliary measurement light is emitted in a direction crossing a visual field of the image pickup element (46), and
a processor (14),
wherein the processor specifies a position of a specific region,
wherein the specific region is a spot (SP) and is a circular shape formed on the subject by the auxiliary measurement light and sets a measurement marker that shows an actual size of the subject according to the position of the specific region and includes gradations of which an end portion serves as a base point, and creates a specific image in which the measurement marker is superimposed on the picked-up image of the subject picked up by the image pickup element so that the position of the specific region and the base point of the gradations of the measurement marker overlap with each other, wherein the end portion is a portion closer to an outer portion than a central portion of the shape of the measurement marker.

2. The endoscope apparatus according to claim 1,
wherein the measurement marker has a shape of a straight line segment or a shape in which straight line segments are combined with each other.

3. The endoscope apparatus according to any one of claims 1 or 2,
wherein the gradations of the measurement marker are uneven.

4. The endoscope apparatus according to any one of claims 1 to 3,
wherein the processor (14) creates the specific image in which the measurement marker is superimposed on the picked-up image so that a center of the measurement marker is positioned closer to a center than the position of the specific region.

5. The endoscope apparatus according to any one of claims 1 to 4,
wherein the auxiliary measurement light source unit (45) emits the auxiliary measurement light so that a trajectory formed by the auxiliary measurement light and a trajectory formed by an optical axis of the image pickup element are different from each other on the subject.

6. The endoscope apparatus according to any one of claims 1 to 5,
wherein the processor (14) receives an instruction to switch the measurement marker to be set, and switches and sets a plurality of the measurement markers, which are different from each other, according to the instruction.

7. The endoscope apparatus according to any one of claims 1 to 6,
wherein the processor (14) switches the measurement marker that includes the gradations of which the end portion serves as a base point to set a measurement marker that includes gradations of which a middle portion serves as a base point.

8. The endoscope apparatus according to any one of claims 1 to 7, further comprising:
an illumination light source unit (41) that emits illumination light used to illuminate the subject; and
an illumination light switch that is used to turn on or off the illumination light,
wherein the auxiliary measurement light source unit does not emit the auxiliary measurement light in a case where the illumination light is turned off by the illumination light switch.

9. A program for an endoscope apparatus including an auxiliary measurement light source unit that emits auxiliary measurement light used for measurement of a subject and an image pickup element that picks up an image of the subject, wherein the auxiliary measurement light is emitted in a direction crossing a visual field of the image pickup element (46), the program causing a computer to execute:
a function of specifying a position of a specific region which is a spot (SP) formed on the subject by the auxiliary measurement light and is a circular shape;
a function of setting a measurement marker that shows an actual size of the subject according to the position of the specific region and includes gradations of which an end portion serves as a base point; and
a function of creating a specific image in which the measurement marker is superimposed on the picked-up image of the subject picked up by the image pickup element so that the position of the specific region and the base point of the gradations of the measurement marker overlap with each other, wherein the end portion is a portion closer to an outer portion than a central portion of the shape of the measurement marker.

## Patentansprüche

1. Endoskopvorrichtung, umfassend:
eine Hilfsmesslicht-Quelleneinheit (45), die Hilfsmesslicht emittiert, das zur Messung eines Motivs verwendet wird;
ein Bildaufnahmeelement (46), das ein Bild des Motivs aufnimmt; wobei das Hilfsmesslicht in einer Richtung emittiert wird, die ein Sichtfeld des Bildaufnahmeelements (46) schneidet, und
einen Prozessor (14),
wobei der Prozessor eine Position eines spezifischen Bereichs spezifiziert,
wobei der spezifische Bereich ein Fleck (SP) ist und eine Kreisform ist, die durch das Hilfsmesslicht an dem Motiv gebildet ist, und eine Messmarkierung, die eine tatsächliche Größe des Motivs zeigt, gemäß der Position des spezifischen Bereichs einstellt und Abstufungen, von denen ein Endabschnitt als ein Basispunkt dient, enthält und ein spezifisches Bild erzeugt, in dem die Messmarkierung dem aufgenommenen Bild des Motivs, das durch das Bildaufnahmeelement aufgenommen wurde, so überlagert ist, dass sich die Position des spezifischen Bereichs und der Basispunkt der Abstufungen der Messmarkierung überlappen, wobei der Endabschnitt ein Abschnitt ist, der näher an einem Außenabschnitt als ein Mittelabschnitt der Form der Messmarkierung liegt.

2. Endoskopvorrichtung nach Anspruch 1,
wobei die Messmarkierung eine Form eines geraden Liniensegments oder eine Form, bei der gerade Liniensegmente miteinander kombiniert sind, aufweist.

3. Endoskopvorrichtung nach Anspruch 1 oder 2,
wobei die Abstufungen der Messmarkierung ungleichmäßig sind.

4. Endoskopvorrichtung nach einem der Ansprüche 1 bis 3,
wobei der Prozessor (14) das spezifische Bild erstellt, in dem die Messmarkierung dem aufgenommenen Bild so überlagert ist, dass eine Mitte der Messmarkierung näher an einer Mitte als der Position des spezifischen Bereichs positioniert ist.

5. Endoskopvorrichtung nach einem der Ansprüche 1 bis 4,
wobei die Hilfsmesslicht-Quelleneinheit (45) das Hilfsmesslicht so emittiert, dass eine Trajektorie, die durch das Hilfsmesslicht gebildet wird, und eine Trajektorie, die durch eine optische Achse des Bildaufnahmeelements gebildet wird, auf dem Motiv voneinander verschieden sind.

6. Endoskopvorrichtung nach einem der Ansprüche 1 bis 5,
wobei der Prozessor (14) eine Anweisung zum Umschalten der einzustellenden Messmarkierung empfängt und mehrere der Messmarkierungen, die voneinander verschieden sind, gemäß der Anweisung umschaltet und einstellt.

7. Endoskopvorrichtung nach einem der Ansprüche 1 bis 6,
wobei der Prozessor (14) die Messmarkierung umschaltet, die die Abstufungen enthält, deren Endabschnitt als ein Basispunkt zum Einstellen einer Messmarkierung dient, die Abstufungen enthält, von denen ein mittlerer Abschnitt als ein Basispunkt dient.

8. Endoskopvorrichtung nach einem der Ansprüche 1 bis 7, ferner umfassend:
eine Beleuchtungslicht-Quelleneinheit (41), die Beleuchtungslicht emittiert, das zum Beleuchten des Motivs verwendet wird;
einen Beleuchtungslichtschalter, der zum Ein- oder Ausschalten des Beleuchtungslichts verwendet wird,
wobei die Hilfsmesslicht-Quelleneinheit das Hilfsmesslicht in einem Fall, in dem das Beleuchtungslicht durch den Beleuchtungslichtschalter ausgeschaltet wird, nicht emittiert.

9. Programm für eine Endoskopvorrichtung, die eine Hilfsmesslicht-Quelleneinheit, die Hilfsmesslicht emittiert, das zur Messung eines Motivs verwendet wird, und ein Bildaufnahmeelement, das ein Bild des Motivs aufnimmt, enthält, wobei das Hilfsmesslicht in einer Richtung emittiert wird, die ein Sichtfeld des Bildaufnahmeelements (46) schneidet, wobei das Programm einen Computer veranlasst, auszuführen:
eine Funktion des Spezifizierens einer Position eines spezifischen Bereichs, der ein Fleck (SP) ist, der durch das Hilfsmesslicht an dem Motiv gebildet ist und eine Kreisform aufweist;
eine Funktion des Einstellens einer Messmarkierung, die eine tatsächliche Größe des Motivs gemäß der Position des spezifischen Bereichs zeigt und Abstufungen, von denen ein Endabschnitt als ein Basispunkt dient, enthält; und
eine Funktion des Erstellens eines spezifischen Bildes, in dem die Messmarkierung dem aufgenommenen Bild des Motivs, das durch das Bildaufnahmeelement aufgenommen wurde, so überlagert ist, dass sich die Position des spezifischen Bereichs und der Basispunkt der Abstufungen der Messmarkierung überlappen, wobei der Endabschnitt ein Abschnitt ist, der näher an einem Außenabschnitt als ein Mittelabschnitt der Form der Messmarkierung liegt.

## Revendications

1. Appareil endoscopique comprenant :
une unité de source de lumière de mesure auxiliaire (45) qui émet de la lumière de mesure auxiliaire utilisée pour la mesure d'un sujet ;
un élément de capture d'images (46) qui capture une image du sujet ; dans lequel la lumière de mesure auxiliaire est émise dans une direction croisant un champ visuel de l'élément de capture d'images (46), et
un processeur (14),
dans lequel le processeur spécifie une position d'une région spécifique,
dans lequel la région spécifique est un point (SP) et a une forme circulaire formée sur le sujet par la lumière de mesure auxiliaire et règle un marqueur de mesure qui montre une taille réelle du sujet en fonction de la position de la région spécifique et inclut des gradations dont une partie d'extrémité sert de point de base, et crée une image spécifique dans laquelle le marqueur de mesure est superposé sur l'image capturée du sujet capturée par l'élément de capture d'images de sorte que la position de la région spécifique et le point de base des gradations du marqueur de mesure se chevauchent,
dans lequel la partie d'extrémité est une partie plus près d'une partie extérieure que une partie centrale de la forme du marqueur de mesure.

2. Appareil endoscopique selon la revendication 1,
dans lequel le marqueur de mesure a une forme d'un segment de ligne droite ou une forme dans laquelle des segments de ligne droite sont combinés entre eux.

3. Appareil endoscopique selon la revendication 1 ou la revendication 2,
dans lequel les gradations du marqueur de mesure sont inégales.

4. Appareil endoscopique selon l'une quelconque des revendications 1 à 3,
dans lequel le processeur (14) crée l'image spécifique dans laquelle le marqueur de mesure est superposé sur l'image capturée de sorte qu'un centre du marqueur de mesure est positionné plus près d'un centre que la position de la région spécifique.

5. Appareil endoscopique selon l'une quelconque des revendications 1 à 4,
dans lequel l'unité de source de lumière de mesure auxiliaire (45) émet la lumière de mesure auxiliaire de sorte qu'une trajectoire formée par la lumière de mesure auxiliaire et une trajectoire formée par un axe optique de l'élément de capture d'images sont différentes l'une de l'autre sur le sujet.

6. Appareil endoscopique selon l'une quelconque des revendications 1 à 5,
dans lequel le processeur (14) reçoit une instruction pour commuter le marqueur de mesure à régler et commute et règle une pluralité des marqueurs de mesure, qui sont différents les uns des autres, en fonction de l'instruction.

7. Appareil endoscopique selon l'une quelconque des revendications 1 à 6,
dans lequel le processeur (14) commute le marqueur de mesure qui inclut les gradations dont la partie d'extrémité sert de point de base pour régler un marqueur de mesure qui inclut des gradations dont une partie médiane sert de point de base.

8. Appareil endoscopique selon l'une quelconque des revendications 1 à 7, comprenant en outre :
une unité de source de lumière d'éclairage (41) qui émet de la lumière d'éclairage utilisée pour éclairer le sujet ; et
un interrupteur de lumière d'éclairage qui est utilisé pour allumer ou éteindre la lumière d'éclairage,
dans lequel l'unité de source de lumière de mesure auxiliaire n'émet pas la lumière de mesure auxiliaire dans un cas où la lumière d'éclairage est éteinte par l'interrupteur de lumière d'éclairage.

9. Programme pour un appareil endoscopique incluant une unité de source de lumière de mesure auxiliaire qui émet de la lumière de mesure auxiliaire utilisée pour mesurer un sujet, et un élément de capture d'images qui capture une image du sujet, dans lequel la lumière de mesure auxiliaire est émise dans une direction croisant un champ visuel de l'élément de capture d'images (46), le programme amenant un ordinateur à exécuter :
une fonction de spécification d'une position d'une région spécifique qui est un point (SP) formé sur le sujet par la lumière de mesure auxiliaire et a une forme circulaire ;
une fonction de réglage d'un marqueur de mesure qui montre une taille réelle du sujet en fonction de la position de la région spécifique et inclut des gradations dont une partie d'extrémité sert de point de base ; et
une fonction de création d'une image spécifique dans laquelle le marqueur de mesure est superposé sur l'image capturée du sujet capturé par l'élément de capture d'images de sorte que la position de la région spécifique et le point de base des gradations du marqueur de mesure se chevauchent, dans lequel la partie d'extrémité est une partie plus près d'une partie extérieure que une partie centrale de la forme du marqueur de mesure.
